# EUROPÄISCHE PATENTSCHRIFT

(11) **EP 2 170 771 B1**
(45) Veröffentlichungstag und Bekanntmachung des Hinweises auf die Patenterteilung: **12.03.2014**
(21) Anmeldenummer: 08773877.9
(22) Anmeldetag: 05.07.2008
(51) Int. Cl.: C02F 1/467, A61K 33/06, C11D 3/48, C11D 7/10, A61K 33/04, C02F 1/461, G03B 15/05, C02F 101/20, C02F 101/30, C02F 103/30, C02F 103/02

(54) **ELEKTROLYSEVERFAHREN MIT DIAPHRAGMA SOWIE ERZEUGNIS UND SEINE VERWENDUNG**
DIAPHRAGM ELECTROLYSIS METHOD, PRODUCTS OBTAINED AND USE OF PRODUCTS
PROCEDE D'ELECTROLYSE AVEC DIAPHRAGME, PRODUITS OBTENUS AINSI QUE LEUR UTILISATION

(30) Priorität: 18.07.2007 DE 102007033445
(43) Veröffentlichungstag der Anmeldung: 07.04.2010
(60) Teilanmeldung: 12169634.8
(73) Patentinhaber: Monopharm Handelsgesellschaft Mbh, 21444 Vierhöfen (DE)
(72) Erfinder: PONITZ, Burkhard, 21444 Vierhöfen (DE)
(74) Vertreter: von Puttkamer · Berngruber
(86) Internationale Anmeldenummer: PCT/EP2008/005501
(87) Internationale Veröffentlichungsnummer: WO 2009/010203

(56) Entgegenhaltungen:
- WO-A-2007/008591
- DE-A1- 19 624 024
- GB-A- 1 184 580
- GB-A- 1 232 521
- US-A- 3 337 433
- US-A- 5 084 149

## Beschreibung

Die Erfindung bezieht sich auf ein Diaphragmalyse-Verfahren nach dem Oberbegriff des Anspruchs 1. Auch hat es die Verwendung der danach erhaltenen Anolyt-Lösungen zum Gegenstand.

Die Diaphragmaanalyse stellt ein bekanntes Wasseraufbereitungsverfahren dar (vgl. EP 1380543 A1, EP 1382573 A1, WO2006/040709 A1). Dazu wird eine Elektrolysezelle verwendet, die einen Anodenraum mit der Anode und einen von dem Anodenraum durch ein Diaphragma getrennten Kathodenraum mit der Kathode aufweist.

Dem Anoden- und Kathodenraum wird eine wässrige Natriumchlorid-Lösung als Elektrolyt zugeführt. Damit bewegen sich die Anionen und sonstige negativ geladene Teilchen in dem Elektrolyt in Richtung der Anode und die Kationen und dergleichen positiv geladene Teilchen in Richtung Kathode, wobei die negativ geladenen Teilchen in dem Kathodenraum bzw. die positiv geladenen Teilchen in dem Anodenraum das Diaphragma passieren müssen, um zu der Anode bzw. Kathode zu gelangen.

Dadurch entsteht in dem Anodenraum eine Anolyt-Lösung, die ein positives Redox-Potential und damit oxidierende Eigenschaften besitzt, während die in dem Kathodenraum gebildete Katholyt-Lösung ein negatives Redox-Potential, also reduzierende Eigenschaften aufweist. Zudem besitzt die Katholyt-Lösung stark basische Eigenschaften, während die Anolyt-Lösung sauer ist.

Während die Anolyt-Lösung, die Natriumhypochlorid, Wasserstoffperoxid, Chlor und Ozon als metastabile Verbindungen enthält, dem zu reinigenden Wasser zudosiert wird, wird die Katholyt-Lösung, die bisher in wesentlich geringerer Menge anfällt als die Anolyt-Lösung, entweder verworfen oder zur Fällung der Schwermetalle eingesetzt (EP 1380543 A1).

Aus US 3,337,433 A ist ein Diaphragmalyse-Verfahren zur Phosphingas-Herstellung bekannt, bei dem als Elektrolyt Natriumchlorid und Natriumsulfat verwendet wird, um die Effizienz der Elektrolysezelle zu erhöhen. Zur Herstellung von Olifenoxiden wird nach GB 1,232,521 A1 ein Diaphragmalyse-Verfahren durchgeführt, bei dem als Elektrolyt eine Alkalichlorid-Lösung verwendet wird, der ein Metallsulfat zugesetzt wird.

Aufgabe der Erfindung ist es, für die Anolyt-Lösung neue Anwendungsgebiete zu erschließen.

Demgemäss wird erfindungsgemäß ein Verfahren bereitgestellt, mit dem die metastabilen oxidierenden Eigenschaften der Anolyt-Lösung stabilisiert werden können, um sie für andere Anwendungsgebiete einsetzen zu können.

Dabei wird für das erfindungsgemäße Verfahren beispielsweise ein im Handel erhältliches Diaphragmalyse-Gerät zur Waseraufbereitung verwendet, bei dem der Durchflussregler auf der Kathodenseite zur Erhöhung der Durchflussmenge des Katholyt umgebaut ist.

Der Elektrolyt besteht vorzugsweise aus Natriumchlorid, er kann jedoch auch aus einem anderen Alkalichlorid gebildet werden. So hat sich insbesondere ein Gemisch aus Natrium- und Kaliumchlorid als geeignet erwiesen, wobei der Kaliumchloridanteil bis zu 50 Gew.%, bezogen auf das Gesamtgewicht der Alkalichloride betragen kann. Die Alkalichlorid-Lösung wird vorzugsweise als gesättigte Lösung aus einem Vorratsbehälter entnommen und mit der gewünschten Menge Wasser, z. B. in einem Volumenverhältnis von 1:5 bis 1:20 verdünnt. Das Natriumsulfat wird vorzugsweise zu der gesättigten Alkalichlorid-Lösung in den Vorratsbehälter gegeben. Das Alkalisulfat wird der Alkalichlorid-Lösung vorzugsweise in einer Menge von 1 bis 50 Gramm, insbesondere 5 bis 20 Gramm/Liter zugesetzt.

Das Wasser, mit dem die Alkalichlorid-Lösung verdünnt wird, ist vorzugsweise ein carbonatfreies oder carbonatarmes Wasser, d. h. der (deutsche) Härtegrad des Wassers beträgt vorzugsweise höchstens 5, also der Calciumcarbonatgehalt höchtens 50 mg/l.

Das Alkalisulfat, das der Alkalichlorid-Lösung zugesetzt wird, ist vorzugsweise Natriumsulfat.

Die Anolyt-Lösung kann eine Redox-Spannung von über +1200 mV und einen pH-Wert von 1 bis 3 aufweisen, die Katholyt-Lösung eine Redox-Spannung von -600 bis -1000 mV und einen pH-Wert von über 12.

Das Ziel der Erfindung, eine stabile Anolyt-Lösung zur Verfügung zu stellen, um für die Anolyt-Lösung neue Anwendungsgebiete erschließen zu können, wird dadurch erreicht, dass der Anolyt-Lösung Aluminium und/oder Magnesiumsulfat zugesetzt wird, und zwar in einer Menge von 0,1 bis 20 Gramm, insbesondere 0,5 bis 5 Gramm, ganz besonders bevorzugt 1 bis 2 Gramm pro Liter Anolyt-Lösung.

Überraschenderweise hat sich gezeigt, dass damit die metastabilen Verbindungen in der Anolyt-Lösung und damit deren Wirksamkeit über Monate derart zuverlässig stabilisiert werden, dass von einer Lagerstabilität der Anolyt-Lösung von 1 bis 2 Jahren ausgegangen werden kann.

Als Aluminiumsulfat zur Stabilisierung der Anolyt-Lösung wird vorzugsweise Kaliumalaun verwendet.

Kaliumalaun, also Kaliumaluminiumsulfat ist in der Naturmedizin als Wirkstoff seit langem bekannt. Es findet z. B. Anwendung im Bereich der Magen-, Darm- und Blasenschwäche, aber auch bei Blutungen. Es ist bekannt, dass Alaun desinfizierend auf Schleimhäute und Wunden wirkt. Gleichzeitig wirkt Alaun allerdings austrocknend, teilweise gerinnend und sekretionsbeschränkend, sodass sein Einsatz in der Medizin eingeschränkt ist.

Es hat sich nun überraschenderweise herausgestellt, dass eine Anolyt-Lösung, die Alaun enthält, eine hervorragende reinigende, dekontaminierende und desinfizierende Wirkung im Mund- und Rachenbereich, ohne nachteilige Folgen auf Zähne, Zahnfüllungen und Zunge, aber auch unter Vermeidung einer Sekretionshemmung und Austrocknung entfaltet. Die erfindungsgemäße, alaunhaltige Anolyt-Lösung ist daher hervorragend als Mundspüllösung geeignet. So können mit ihr auch hartnäckigste Entzündungen im Mund- und Rachenbereich erfolgreich behandelt werden. Blutungen, die z. B. bei der zahnärztlichen Behandlung auftreten, werden innerhalb weniger Minuten gestoppt. Weiterhin kann die Mundspüllösung prophylaktisch eingesetzt werden, um die Entstehung von Parodontopathien, Gingivitis und Stomatitis zu vermeiden.

Die Mundspüllösung kann weiterhin Magnesiumsulfat enthalten, wodurch die Lagerstabilität weiter erhöht wird. Die Menge des Magnesiumsulfats kann beispielsweise 0,5 bis 10 Gramm pro Liter Anolyt-Lösung, insbesondere 1 bis 2 Gramm pro Liter betragen.

Die Mundspüllösung kann weitere allgemein übliche Zusatzstoffe enthalten, beispielsweise Aromastoffe, Süssstoffe, ätherische Öle und dergleichen.

Es hat sich gezeigt, dass die erfindungsgemäße Mundspüllösung Keime, die für das Entstehen von Plaque, Gingivitis und Parodontitis verantwortlich sind, zuverlässig beseitigt. Infektionen werden so wirksam vorgebeugt und eventuell vorhandene Wunden heilen beschleunigt ab. Ebenso wenig treten Verfärbungen des Zahnfleisches, der Zähne oder der Zunge auf.

Die erfindungsgemäße Anolyt-Lösung ist jedoch nicht nur als Mundspüllösung verwendbar, vielmehr ist sie auf den verschiedensten Indikationsgebieten einsetzbar und zwar sowohl in der Human- wie in der Tiermedizin. So besitzt sie eine hohe biozide, insbesondere antibakterielle und fungizide Wirkung.

So kann die erfindungsgemäße Anolyt-Lösung zur Wundbehandlung, beispielsweise bei Hautverletzungen oder zur Behandlung von Geschwüren eingesetzt werden. Insbesondere hat sich die erfindungsgemäße Anolyt-Lösung hervorragend zur Behandlung des Magen-Darm-Trakts erwiesen, um beispielsweise durch Heliobacter oder Candida hervorgerufene Krankheiten oder Blutungen zu beseitigen. Neben einer starken antibakteriellen Wirkung weist die erfindungsgemäße Anolyt-Lösung zudem eine stark fungizide Wirkung auf. Damit können sowohl beim Menschen Pilzinfektionen, beispielsweise Nagelpilz erfolgreich bekämpft werden, wie z. B. die Huffäule, Mauke oder der Sattelpilz bei Pferden.

Darüber hinaus kann die erfindungsgemäße Anolyt-Lösung zur Behandlung von Ekzemen, beispielsweise dem Sommerekzem bei Pferden eingesetzt werden, aber auch zur Bekämpfung der Stirnhöhlenvereiterung z. B. bei Pferden, sowie von Entzündungen und Infektionen bei Tier und Mensch, bis hin zur Zeckenprophylaxe. Dabei ist die erfindungsgemäße Anolyt-Lösung insbesondere auch bei Krankheiten einsetzbar, die aufgrund von Antibiotikaresistenzen nicht mehr behandelt werden können.

Ein weiteres Anwendungsgebiet der erfindungsgemäßen Analyt-Lösung stellt die Haltbarmachung von Cremes, beispielsweise zur Körperpflege dar. Denn neben der desinfizierenden Wirkung weist die erfindungsgemäße Anolyt-Lösung zugleich eine stark emulgierende Wirkung auf. Zudem hat sie gegenüber anderen in Cremes verwendeten Zusatzstoffen den Vorteil, dass sie zu keinen Allergien führt.

Weiterhin hat sich herausgestellt, dass sich die erfindungsgemäße Anolyt-Lösung hervorragend als Trinkwasserzusatz bei der Geflügelhaltung eignet.

So wurden letztes Jahr, als aufgrund der Vogelgrippe die Hühner aufgestallt werden mussten, in einer Hühnerfarm mit mehreren 100 Hühnern, die eine Hälfte mit Trinkwasser versorgt, welches die erfindungsgemäße Anolyt-Lösung in einer Verdünnung von 1 Volumenteil Anolyt-Lösung zu zehn Volumenteilen Wasser in der ersten Woche und in einer Verdünnung von 1:100 in den darauf folgenden fünf Wochen enthielt, während die andere Hälfte der Hühner als Kontrollgruppe das Trinkwasser ohne die Anolyt-Lösung verabreicht bekam. Nach sechs Wochen wurde eine Gewichtszunahme bei der Hühnern, die die Anolyt-Lösung erhielten, von über 30 % gegenüber der Kontrollgruppe festgestellt.

Außer zur Geflügelhaltung ist die Anolyt-Lösung generell in der Tierzucht als Trinkwasserzusatz geeignet.

Zudem ist die erfindungsgemäße Anolyt-Lösung hervorragend als Reinigungsmittel z.B. im Lebensmittelbereich geeignet. So kann sie beispielsweise zur Reinigung von Leitungen, Behältern oder dergleichen z.B. in Brauereien oder Molkereien oder generell zur Reinigung von Anlagen zur Nahrungsmittel- einschließlich Getränkeproduktion eingesetzt werden. Dazu gehört insbesondere auch die Herstellung Kühleis. So ist Kühleis, das mit einer mit der erfindungsgemäßen Anolyt-Lösung gereinigte Anlage hergestellt worden ist, aufgrund seiner Keimfreiheit in direktem Kontakt auch mit leicht verderblichen Lebensmitteln, wie Fischen, mit großen Erfolg eingesetzt worden.

Das heißt, die erfindungsgemäß hergestellte Anolyt-Lösung ist generell zur Reinigung von Oberflächen im Nahrungsmittelbereich, einschließlich Getränke, verwendbar. Insbesondere kann sie zur Reinigung von Anlagen oder Gegenständen, die zur Herstellung von Nahrungsmitteln eingesetzt werden, oder von Hilfsmitteln, wie Kühleis, die mit Nahrungsmitteln in Kontakt kommen, verwendet werden.

Die erfindungsgemäße Anolyt-Lösung besitzt eine hohe reinigende Wirkung und ist daher generell zur Herstellung von Reinigungsmitteln verwendbar.

## Patentansprüche

1. Diaphragmalyse-Verfahren, bei dem dem Anodenraum und dem Kathodenraum eine Elektrolysezelle, die durch ein Diaphragma voneinander getrennt sind, jeweils eine wässrige Alkalichlorid-Lösung zugeführt wird, um eine Anolyt-Lösung und eine Katholyt-Lösung zu bilden, **dadurch gekennzeichnet, dass** der zugeführten Alkalichlorid-Lösung zur Erhöhung der Katholyt-Produktion 1 bis 50 Gramm Alkalisulfat pro Liter zugesetzt werden und der Anolyt-Lösung Aluminium- und/oder Magnesiumsulfat in einer Menge von 0,1 bis 20 Gramm/Liter zugesetzt wird.

2. Verfahren nach Anspruch 1, **dadurch gekennzeichnet, dass** das Alkalisulfat Natriumsulfat ist.

3. Verfahren nach einem der vorstehenden Ansprüche, **dadurch gekennzeichnet, dass** die zugeführte wässrige Alkalichlorid-Lösung weniger als 50 mg Calciumcarbonat pro Liter enthält.

4. Verfahren nach einem der Ansprüche 1 bis 3, **dadurch gekennzeichnet, dass** als Aluminiumsulfat Alaun verwendet wird.

5. Anolyt-Lösung, erhalten durch das Verfahren nach einem der vorstehenden Ansprüche, zur Anwendung in der Human- und/oder Tiermedizin.

6. Anolyt-Lösung nach Anspruch 5 zur Wundbehandlung.

7. Anolyt-Lösung nach Anspruch 5 zur Behandlung des Magen-Darm-Trakts.

8. Anolyt-Lösung nach Anspruch 5 zur Prophylaxe und/oder Therapie von Pilzerkrankungen.

9. Anolyt-Lösung nach Anspruch 5 zur Behandlung von Ekzemen und/oder Infektionen.

10. Anolyt-Lösung, erhalten durch das Verfahren nach einem der Ansprüche 1 bis 4, zur Haltbarmachung von Cremes.

11. Verwendung der nach einem der Ansprüche 1 bis 4 erhaltenen Anolyt-Lösung zur Herstellung eines Trinkwasserzusatzes in der Geflügelhaltung oder Tierzucht.

12. Verwendung der nach einem der Ansprüche 1 bis 4 erhaltenen Anolyt-Lösung als Reinigungsmittel im Lebensmittelbereich.

## Claims

1. Diaphragmalysis procedure in which an aqueous alkali-chloride solution is added to both the anodic region and the cathode region of an electrolytic cell which are separated by a diaphragm in order to form an anolyte solution, and a catholyte solution that is **characterised in that** 1 to 50 gram of alkali sulphate per litre is added to the added alkali-chloride solution in order to increase catholyte production, and 0.1 to 20 gram of aluminium and/or magnesium sulphate per litre is added to the anolyte solution.

2. A method according to claim 1, **characterised by** the alkali sulphate being sodium sulphate.

3. A method according to one of the above claims, **characterised in that** the aqueous alkalichloride solution added contains less than 50 mg of calcium carbonate per litre.

4. A method according to one of the above claims 1 to 3, **characterised in that** the aluminium sulphate used is alum.

5. An anolyte solution obtained by the method of one of the above claims for use in human and/or veterinary medicine.

6. An anolyte solution according to claim 5 used for dressing wounds.

7. An anolyte solution according to claim 5 used for treating the gastrointestinal tract.

8. An anolyte solution according to claim 5 used for prophylaxis and/or treating mycosis.

9. An anolyte solution according to claim 5 used for treating eczemas and/or infections.

10. An anolyte solution obtained by the method of one of the claims 1 to 4 for the preservation of lotions.

11. Use of the anolyte solution obtained by the method of one of the claims 1 to 4 for preparing an additive for drinking water in poultry farming or animal breeding.

12. Use of the anolyte solution obtained by the method of one of the claims 1 to 4 as a cleaning agent in food areas.

## Revendications

1. Procédé d'électrolyse avec diaphragme dans lequel une solution aqueuse de chlorure alcalin est acheminée respectivement au compartiment d'anode et au compartiment de cathode d'une cellule d'électrolyse, qui sont séparés l'un de l'autre par un diaphragme, pour former une solution d'anolyte et une solution de catholyte, **caractérisé en ce qu'** à la solution de chlorure alcalin acheminée, on ajoute 1 à 50 grammes de sulfate alcalin par litre pour augmenter la production de catholyte et **en ce qu'**à la solution d'anolyte, on ajoute du sulfate d'aluminium et/ou de magnésium dans une quantité de 0,1 à 20 grammes par litre.

2. Procédé selon la revendication 1, **caractérisé en ce que** le sulfate alcalin est du sulfate de sodium.

3. Procédé selon l'une des revendications précédentes, **caractérisé en ce que** la solution aqueuse de chlorure alcalin contient moins de 50 mg de carbonate de calcium par litre.

4. Procédé selon l'une des revendications 1 à 3, **caractérisé en ce qu'**on utilise de l'alun en tant que sulfate d'aluminium.

5. Solution d'anolyte, obtenue par le procédé selon l'une des revendications précédentes, destinée à être utilisée en médecine humaine ou vétérinaire.

6. Solution d'anolyte selon la revendication 5, destinée au traitement de plaies.

7. Solution d'anolyte selon la revendication 5, destinée au traitement du système gastro-intestinal.

8. Solution d'anolyte selon la revendication 5, destinée à la prophylaxie et/ou à la thérapie de maladies fongiques.

9. Solution d'anolyte selon la revendication 5, destinée au traitement d'eczémas et/ou d'infections.

10. Solution d'anolyte obtenue par le procédé selon l'une des revendications 1 à 4, destinée à la conservation de crèmes.

11. Utilisation de la solution d'anolyte obtenue selon l'une des revendications 1 à 4, destinée à la fabrication d'un complément à l'eau de boisson dans l'élevage de volailles ou dans la production animale.

12. Utilisation de la solution d'anolyte obtenue selon l'une des revendications 1 à 4 en tant que produit de nettoyage dans le domaine alimentaire.
